**Europäisches Patentamt**

(19) **European Patent Office**

**Office européen des brevets**

(11) Veröffentlichungsnummer: **0 037 913**
**B1**

(12) # EUROPÄISCHE PATENTSCHRIFT

(45) Veröffentlichungstag der Patentschrift:
**13.02.85**

(51) Int. Cl.⁴: **A 61 B 6/02,** H 01 R 39/50,
H 05 G 1/10

(21) Anmeldenummer: **81101990.0**

(22) Anmeldetag: **17.03.81**

(54) **Röntgengerät zur Herstellung von Transversal-Schichtbildern.**

(30) Priorität: **20.03.80 DE 3010819**

(43) Veröffentlichungstag der Anmeldung:
**21.10.81 Patentblatt 81/42**

(45) Bekanntmachung des Hinweises auf die Patenterteilung:
**13.02.85 Patentblatt 85/7**

(84) Benannte Vertragsstaaten:
**DE FR GB**

(56) Entgegenhaltungen:
**CH - A - 364 912**
**DE - A - 2 716 818**
**FR - A - 2 333 237**
**GB - A - 1 338 869**
**US - A - 3 136 590**
**US - A - 4 181 850**

(73) Patentinhaber: **Siemens Aktiengesellschaft, Berlin und München Wittelsbacherplatz 2, D-8000 München 2 (DE)**

(72) Erfinder: **Distler, Walter, Sudetenlandstrasse 23a, D-8520 Erlangen-Dechsendorf (DE)**
Erfinder: **Heinzelmann, Karl-Georg, Weingasse 61, D-8524 Neunkirchen (DE)**

**Beschreibung**

Die Erfindung betrifft ein Röntgenschichtgerät zur Herstellung von Transversalschichtbildern eines Aufnahmeobjektes mit einer Patientenliege, mit einer Röntgenstrahlen-Meßanordnung mit einer von einem Röntgengenerator gespeisten Röntgenstrahlenquelle, die ein das Aufnahmeobjekt durchdringendes Röntgenstrahlenbündel erzeugt, und mit einem Strahlenempfänger, der die Strahlenintensität hinter dem Objekt ermittelt, mit einer Antriebsvorrichtung für die Meßanordnung mit einem Drehrahmen zur Erzeugung von Drehbewegungen der Meßanordnung und mit einem Meßwertumformer für die Transformation der vom Strahlenempfänger gelieferten Signale in ein Schichtbild, bei dem die Stromversorgung der Röntgenröhre über zwischen dem Drehrahmen und dem feststehenden Geräteteil angeordnete Schleifringe mit Gleitkontakten erfolgt, und eine zentrische Öffnung für das Aufnahmeobjekt vorgesehen ist.

Ein Röntgenschichtgerät dieser Art ist beispielsweise in der DE-A-2 716 818 beschrieben. Es wird als Computertomograph bezeichnet und erlaubt aufgrund der Verwendung der Schleifringe eine Dauerrotation der Meßanordnung und damit eine sehr schnelle Abtastung des Aufnahmeobjekts unter verschiedenen Richtungen. Der Meßwertumformer berechnet aus den bei unterschiedlichen Projektionen gebildeten Ausgangssignalen des Strahlenempfängers die Schwächungskoeffizienten vorbestimmter Punkte der untersuchten Transversalschicht. Die berechneten Schwächungskoeffizienten können in Form eines Schichtbildes auf einem Fernsehsichtgerät wiedergegeben werden.

Bei dem bekannten Computertomographen sitzen auf einer Achse mehrere Tragkörper für die Schleifringe, an denen die Gleitkontakte in radialer Richtung anliegen. Die Schleifringe und die Gleitkontakte sind dabei in einem mit Isolieröl gefüllten Behälter angeordnet. Die Verwendung von Isolieröl bedeutet jedoch einen relativ großen Aufwand, insbesondere auch deshalb, weil dieses Öl von Zeit zu Zeit gewechselt werden muß. Ferner ist bei dem bekannten Computertomographen die Bewegungsmöglichkeit des Aufnahmeobjekts in Liegenlängsrichtung begrenzt, da die Schleifringanordnung mit den Gleitkontakten am einen Ende des Computertomographen vorgesehen ist und dort den Einschubweg begrenzt.

Der Erfindung liegt die Aufgabe zugrunde, ein Röntgenschichtgerät der eingangs genannten Art so auszubilden, daß einerseits auf die Verwendung von Isolieröl verzichtet werden kann und andererseits ein freies Hindurchschieben des Aufnahmeobjekts durch den Computertomographen möglich ist.

Diese Aufgabe wird gemäß den Merkmalen des kennzeichnenden Teils des Anspruchs 1 gelöst.

Aufgrund der Tatsache, daß die die Kontaktanordnung tragenden Körper als koaxiale Ringe ausgebildet sind, ist es möglich, das Aufnahmeobjekt axial durch diese Ringe hindurchzuschieben, so daß das Aufnahmeobjekt frei einstellbar ist.

Es ist besonders zweckmäßig, insgesamt drei Schleifringe zur Zuführung der Hoch- und der Heizspannung zur Röntgenröhre vorzusehen, von denen die beiden zur Kathode führenden Schleifringe mit den zugeordneten Gleitkontakten in einer Kammer und der zur Anode führende Schleifring mit dem zugeordneten Gleitkontakt in einer zweiten Kammer liegen, wobei zwischen den Kammern jeweils ineinandergreifende Rippen der koaxialen Ringe liegen. Bei dieser Ausführung dienen die beiden in einer Kammer liegenden Schleifringe mit den zugeordneten Gleitkontakten zur Zuführung des Kathodenpotentials und der Heizspannung und der andere Schleifring mit dem zugeordneten Gleitkontakt zur Zuführung des Anodenpotentials.

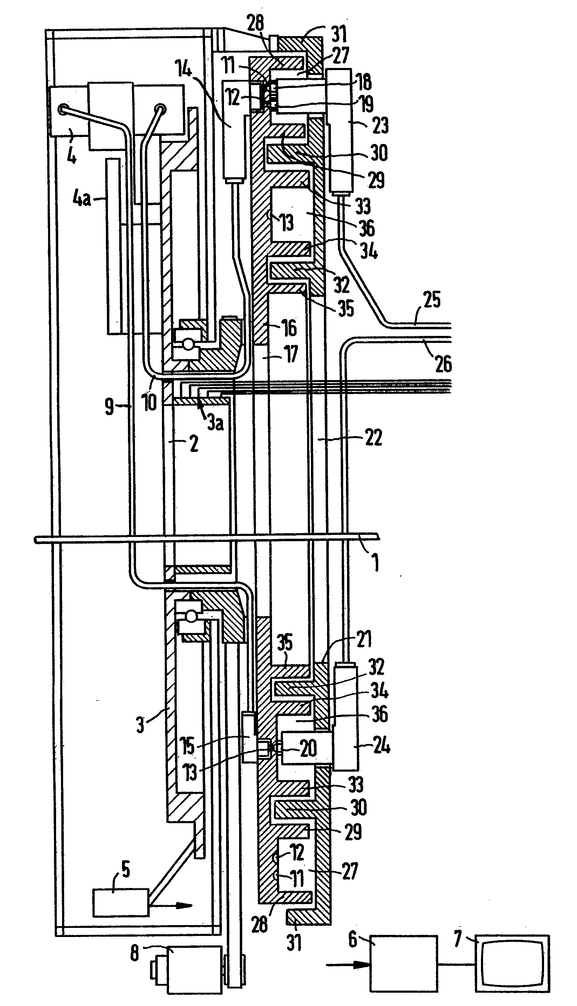

Die Erfindung ist nachfolgend anhand eines in der Zeichnung im Querschnitt dargestellten Ausführungsbeispiels näher erläutert.

In der Zeichnung ist ein Röntgenschichtgerät zur Herstellung von Transversalschichtbildern eines Aufnahmeobjekts mit einer Patientenliege 1 dargestellt, die in der zentrischen Öffnung 2 eines Drehrahmens 3 liegt. Am Drehrahmen 3 sind eine Röntgenröhre 4 und ein Strahlenempfänger 5 befestigt. Die Röntgenröhre 4 sendet ein durch eine Blende 4a eingeblendetes fächerförmiges Röntgenstrahlenbündel aus, das einen Objektkreis mit seinen Randstrahlen tangiert und auf einem Detektor-Array auftrifft, das den Strahlenempfänger 5 bildet und aus einer Reihe von Einzeldetektoren, beispielsweise 512 Einzeldetektoren, besteht. Wird das Aufnahmeobjekt, das auf der Liege 1 liegt, unter verschiedenen Projektionen vom Röntgenstrahlenbündel abgetastet, so kann aus den Ausgangssignalen des Strahlenempfängers 5 durch einen Meßwertumformer 6, der einen Computer enthält, ein Bild der untersuchten Transversalschicht des Aufnahmeobjekts in Form einer Matrix von Schwächungskoeffizienten berechnet und auf einem Sichtgerät 7 wiedergegeben werden. Der Drehrahmen 3 wird von einem Antriebsmotor 8 zur Änderung der Durchstrahlungsrichtung angetrieben.

Die Stromversorgung der Röntgenröhre 4 erfolgt über Hochspannungskabel 9 und 10, die zu drei Schleifringen 11, 12 und 13 über Steckvorrichtungen 14 und 15 geführt sind. Die Schleifringe 11, 12 und 13 liegen konzentrisch zueinander auf einem Ring 16 aus Isoliermaterial, der eine zentrische Öffnung 17 für das Aufnahmeobjekt und die Patientenliege 1 aufweist und mit dem Drehrahmen 3 fest verbunden ist. Die den Schleifringen 11 und 13 zugeordneten ortsfesten Gleitkontakte 18, 19 und 20 sind an einem ortsfesten Ring 21 aus Isoliermaterial angeordnet, der ebenfalls eine zentrische Öffnung 22 für die Patientenliege 1 und das Aufnahmeobjekt besitzt. Die Gleitkontakte 18, 19, 20 sind in Steckern 23 und 24 federnd gelagert. Von den Steckern 23 und 24 führen Hochspannungskabel 25 und 26 zum Hochspannungs- und Heizspannungserzeuger für die Röntgenröhre 4.

Die Schleifringe 11 und 12 liegen in einer Kammer 27, die von zwei Rippen 28 und 29 des drehbaren Ringes 16 begrenzt wird. Der feststehende Ring 21 besitzt Rippen 30 und 31 sowie eine weitere Rippe 32. Ferner besitzt auch der drehbare Ring 16 noch weitere Rippen 33, 34 und 35. Die Rippen der Ringe 16 und 21 greifen labyrinthartig ineinander, wobei die Rippen 29 und 33 sowie 34 und 35 des Ringes 16 Nuten für die Rippen 30 und 32 des Ringes 21 bilden. Dadurch ergeben sich Kriechwege solcher Länge, daß eine Luftisolierung der Schleifringe 11 bis 13 mit den zugeordneten Schleifkontakten 18 bis 20 ausreicht.

Zusammenfassend ist somit als wesentliches Merkmal des Ausführungsbeispiels festzuhalten, daß die Ringe 16 und 21 mit axial vorstehenden Rippen und dazu passenden Nuten ineinandergreifen und deren zentrische Öffnungen zum Einführen der Patientenliege 1 ausgebildet sind.

Bei dem Ausführungsbeispiel sind insgesamt drei Schleifringe 11, 12 und 13 vorgesehen, von denen die Schleifringe 11 und 12 zusammen mit den zugeordneten Gleitkontakten 18 und 19 in einer von den Rippen 28 und 29 begrenzten Kammer 27 liegen und zur Kathode der Röntgenröhre 4 führen und dieser das Kathodenpotential sowie die Heizspannung zuführen. Der Schleifring 13 dient zur Zuführung des Anodenpotentials zur Röntgenröhre 4 und liegt zusammen mit dem ihm zugeordneten Gleitkontakt 20 in einer Kammer 36, die von den Rippen 33 und 34 begrenzt ist. Zwischen den Kammern 27, 36 liegen dabei jeweils ineinandergreifende Rippen, so daß Kriechströme bei der Verwendung von Luft als Isolierstoff praktisch ausgeschlossen sind.

Die Signalübertragung vom Strahlenempfänger 5 zum Meßwertumformer 6 kann ebenfalls über eine Schleifringanordnung erfolgen. Hier treten jedoch keine Isolationsprobleme auf, da nur Niederspannungen zu übertragen sind. Diese Schleifringanordnung 3a ist nur schematisch dargestellt.

**Patentansprüche**

1. Röntgenschichtgerät zur Herstellung von Transversalschichtbildern eines Aufnahmeobjektes mit einer Patientenliege (1), mit einr Röntgenstrahlen-Meßanordnung (4, 5) mit einer von einem Röntgengenerator gespeisten Röntgenstrahlenquelle (4), die ein das Aufnahmeobjekt durchdringendes Röntgenstrahlenbündel erzeugt, und mit einem Strahlenempfänger (5), der die Strahlenintensität hinter dem Objekt ermittelt, mit einer Antriebsvorrichtung (8) für die Meßanordnung mit einem Drehrahmen (3) zur Erzeugung von Drehbewegungen der Meßanordnung und mit einem Meßwertumformer (6) für die Transformation der vom Strahlenempfänger (5) gelieferten Signale in ein Schichtbild, bei dem die Stromversorgung der Röntgenröhre (4) über zwischen dem Drehrahmen (3) und dem feststehenden Geräteteil angeordnete Schleifringe (11, 12, 13) mit Gleitkontakten (18, 19, 20) erfolgt, und eine zentrische Öffnung (2) für das Aufnahmeobjekt vorgesehen ist, dadurch gekennzeichnet, daß die Schleifringe (11, 12, 13) konzentrisch zueinander auf einem Isolierstoffring (16) und die Gleitkontakte (18, 19, 20) auf einem dazu koaxialen, zweiten Isolierstoffring (16, 21) vorgesehen sind, von denen einer (16) mit dem Drehrahmen (3) verbunden und der andere ortsfest ist, die mit axial vorstehenden Rippen (28 bis 35) ineinandergreifen, wobei die Öffnungen (17, 22) der Isolierstoffringe (16, 21) zentrisch angeordnet sind und die Patientenliege (1) durch diese durchschiebbar ist.

2. Röntgenschichtgerät nach Anspruch 1, dadurch gekennzeichnet, daß die Schleifringe (11, 12, 13) am drehbaren Ring (16) und die Gleitkontakte (18, 19, 20) am ortsfesten Ring (21) angeordnet sind.

3. Röntgenschichtgerät nach Anspruch 1 oder 2, dadurch gekennzeichnet, daß insgesamt drei Schleifringe (11, 12, 13) zur Zuführung der Hoch- und der Heizspannung zur Röntgenröhre (4) vorhanden sind, von denen die beiden zur Kathode führenden Schleifringe (11, 12) mit den zugeordneten Gleitkontakten (18, 19) in einer Kammer (27) und der zur Anode führende Schleifring (13) mit dem zugeordneten Gleitkontakt (20) in einer zweiten Kammer (36) liegen, wobei zwischen den Kammern (27, 36) ineinandergreifende Rippen (29, 30, 33) der koaxialen Ringe (16, 21) liegen.

**Claims**

1. An X-ray apparatus for the production of transversal tomographs of an object to be photographed, with a patient's bed (1), an X-ray measuring arrangement (4, 5), an X-ray source (4) fed by an X-ray generator and producing a beam of X-rays which penetrate the objet to be photographed, an X-ray receiver (5) which determined the X-ray intensity behind the object, a drive device (8) for the measuring arrangement with a rotating frame (3) which serves to produce rotational movements of the measuring arrangment, a measured value transformer (6) which serves to transform the signal supplied by the X-ray receiver (5) into a tomograph, slip rings (11, 12, 13) arranged between the rotating frame (3) and a stationary component to supply the X-ray tube (4) with current, sliding contacts (18, 19, 20), and a central opening (2) provided for the object which is to be photographed, characterised in that the slip rings (11, 12, 13) are arranged concentrically with one another on an insulating ring (16), and the sliding contacts (18, 19, 20) are arranged on a second insulating ring (16, 21) coaxial with the first, of which one ring (16) is connected to the rotating frame and the other is stationary, where said rings engage with one another by means of axially projecting ribs (28—35), and where the openings (17, 22) in the insulating rings (16, 21) are centrally arranged so the patient's bed (1) can be pushed through said openings.

2. An X-ray device as claimed in Claim 1, characterised in that the slip rings (11, 12, 13) are arranged on the rotatable ring (16) and the sliding contacts (18, 19, 20) are arranged on the stationary ring (21).

3. An X-ray device as claimed in Claim 1 or 2, characterised in that a total of three slip rings (11, 12, 13) are provided for the supply of the high-voltage and heating voltage to the X-ray tube (4), of which the two slip rings (11, 12) which lead to the cathode are arranged with the assigned sliding contacts (18, 19) in one chamber (27), and the slip ring (13) which leads to the anode is arranged with the assigned sliding contact (20) in a second chamber (36), interengaging ribs (29, 30, 33) of the coaxial rings (16, 21) being arranged between the chambers (27, 36).

## Revendications

1. Appareil de tomographie servant à réaliser des tomographies transversales d'un objet de prise de vue comportant une couchette pour patient (1), un dispositif (4, 5) de mesure du rayonnement X, comportant une source (4) de rayons X alimentée par un générateur radiologique et qui produit un faisceau de rayons X traversant l'objet de prise de vue, et un récepteur de rayonnement (5) qui détermine l'intensité du rayonnement derrière l'objet, un dispositif d'entraînement (8) pour le dispositif de mesure et comportant un cadre rotatif (3) servant à produire des mouvements de rotation du dispositif de mesure, et un transformateur de valeurs de mesure (6) servant à transformer les signaux délivrés par le récepteur de rayonnement (5) en une tomographie, et dans lequel l'alimentation en courant du tube (4) à rayons X est réalisée par l'intermédiaire d'anneaux coulissants (11, 12, 13) disposés entre le cadre rotatif (3) et la partie fixe de l'appareil, avec des contacts glissants (18, 19, 20), et dans lequel il est prévu une ouverture centrée (2) pour l'objet de prise de vues, caractérisé en ce que les anneaux coulissants (11, 12, 13) sont prévus concentriquement les uns aux autres sur un anneau en matériau isolant (16) et les contacts glissants (18, 19, 20) sont prévus sur un second anneau en matériau isolant (16, 21), coaxial au premier, que l'un (16) des anneaux est relié au cadre rotatif (3) et que l'autre est monté fixe, et que ces anneaux engrènent mutuellement par l'intermédiaire de nervures en saillie axiale (28 à 35), les ouvertures (17, 22) des anneaux en matériau isolant (16, 21) étant disposées en position centrée et la couchette (1) pour patient pouvant être insérée à travers ces anneaux.

2. Appareil de tomographie suivant la revendication 1, caractérisé par le fait que les anneaux coulissants (11, 12, 13) sont montés sur l'anneau rotatif (16) et que les contacts glissants (18, 19, 20) sont montés sur l'anneau fixe (21).

3. Appareil de tomographie suivant la revendication 1 ou 2, caractérisé par le fait que pour l'amenée de la haute tension et de la tension de chauffage au tube (4) à tayons X, il est prévu au total trois anneaux coulissants (11, 12, 13), parmi lesquels les deux anneaux coulissants (11, 12) ainsi que les contacts coulissants associés (18, 19) sont situés dans une chambre (27) et que l'anneau coulissant (13) aboutissant à l'anode ainsi que le contact glissant associé (20) sont situés dans une seconde chambre (36), tandis que des nervures (22, 30, 33), des anneaux coaxiaux (16, 21) qui s'interpénètrent, sont situées entre les chambres (27, 36).